(19) Europäisches Patentamt
European Patent Office
Office européen des brevets

(11) Veröffentlichungsnummer : **0 544 151 B1**

(12) # EUROPÄISCHE PATENTSCHRIFT

(45) Veröffentlichungstag der Patentschrift :
**15.06.94 Patentblatt 94/24**

(51) Int. Cl.$^{5}$ : **C07D 213/82,** A01N 43/40

(21) Anmeldenummer : **92119333.0**

(22) Anmeldetag : **12.11.92**

(54) **Hydroxypyridoncarbonsäureamide, deren Herstellung und Verwendung als Herbizide.**

(30) Priorität : **26.11.91 DE 4138819**

(43) Veröffentlichungstag der Anmeldung :
**02.06.93 Patentblatt 93/22**

(45) Bekanntmachung des Hinweises auf die Patenterteilung :
**15.06.94 Patentblatt 94/24**

(84) Benannte Vertragsstaaten :
**BE CH DE FR GB IT LI NL**

(56) Entgegenhaltungen :
**EP-A- 0 104 876**
**EP-A- 0 292 990**

(73) Patentinhaber : **BASF Aktiengesellschaft**
**Carl-Bosch-Strasse 38**
**D-67063 Ludwigshafen (DE)**

(72) Erfinder : **Theobald, Hans, Dr.**
**Queichstrasse 6**
**W-6703 Limburgerhof (DE)**
Erfinder : **von Deyn, Wolfgang, Dr.**
**Luederitzstrasse 4**
**W-6730 Neustadt (DE)**
Erfinder : **Nuebling, Christoph, Dr.**
**Wilhelmstrasse 13**
**W-6733 Hassloch (DE)**
Erfinder : **Walter, Helmut, Dr.**
**Gruenstadter Strasse 82**
**W-6719 Obrigheim (DE)**
Erfinder : **Kardorff, Uwe, Dr.**
**D 3,4**
**W-6800 Mannheim 1 (DE)**
Erfinder : **Westphalen, Karl-Otto, Dr.**
**Mausbergweg 58**
**W-6720 Speyer (DE)**
Erfinder : **Kappe, Thomas, Dr.**
**Obere Teichstrasse 19e**
**A-8010 Graz (AT)**
Erfinder : **Gerber, Matthias, Dr.**
**Brandenburgerstrasse 24**
**W-6703 Limburgerhof (DE)**

EP 0 544 151 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Hydroxypyridoncarbonsäureamide der allgemeinen Formel I,

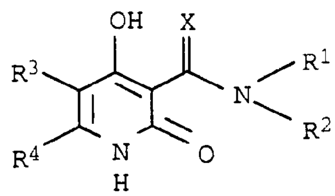

(I),

in der die Substituenten folgende Bedeutungen haben:

X Sauerstoff oder Schwefel;

$R^1$, $R^2$ unabhängig voneinander Wasserstoff, verzweigtes oder unverzweigtes $C_1$-$C_{25}$-Alkyl, $C_3$-$C_{25}$-Alkenyl, $C_3$-$C_{25}$-Alkinyl, $C_3$-$C_8$-Cycloalkyl, $C_3$-$C_8$-Cycloal- kyl-$C_1$-$C_{17}$-alkyl, $C_1$-$C_{25}$-Alkoxy, wobei die genannten organischen Reste gegebenenfalls substituiert sein können durch Halogen, Cyano, $C_1$-$C_5$-Alkylcarbonyl, $C_1$-$C_5$-Alkoxycarbonyl, $C_1$-$C_6$-Alkoxy, $C_1$-$C_6$-Halogenalkoxy, $C_1$-$C_6$-Alkylthio, $C_1$-$C_6$-Halogenalkylthio, gegebenenfalls substituiertes Phenyl, gegebenenfalls substituiertes Heterocyclyl oder durch einen gegebenenfalls substituierten heteroaromatischen Rest;

$R^3$ Wasserstoff, $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen oder die Nitrogruppe;

$R^4$ $C_1$-$C_{10}$-Alkyl, $C_2$-$C_{10}$-Alkenyl, $C_2$-$C_{10}$-Alkinyl, $C_3$-$C_{10}$-Cycloalkyl, $C_1$-$C_{10}$-Alkoxy, $C_1$-$C_{10}$-Alkylthio, Phenyl oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten Reste gegebenenfalls substituiert sind durch Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Halogenalkylthio, gegebenenfalls substituiertes Phenyl oder einen gegebenenfalls substituierten heterocyclischen Rest wie vorstehend genannt;

und deren Salze; ausgenommen 6-Methyl-4-hydroxy-pyrid-2-one-3-carbonsäureamid und 6-Methyl-4-hydroxy-pyrid-2-on-3-carbonsäure-tert-butylamid.

Außerdem betrifft die Erfindung die Herstellung der Verbindungen I und ihre Verwendung als Herbizide.

Es sind bereits 2-Hydroxypyridoncarbonsäureamide in der Literatur bekannt, nämlich 6-Methyl-4-hydroxypyrid-2-on-3-carbonsäureamid (EP-A-104876) und 6-Methyl-4-hydroxypyrid-2-on-3-carbonsäure-tert-butylamid (Dissertation Ahmed Fahmy Ali Khattab, Universität Graz, 1990). Herbizide Wirkungen dieser Verbindungen sind jedoch nicht beschrieben.

Der Erfindung lag die Aufgabe zugrunde, Hydroxypyridoncarbonsäureamide mit guter herbizider Wirkung bereitzustellen. Demgemäß wurden die eingangs beschriebenen Verbindungen, sowie Verfahren zu deren Herstellung und herbizide Mittel, enthaltend diese Verbindungen gefunden.

Die Hydroxypyridoncarbonsäureamide I können in folgenden tautomeren Formen vorliegen:

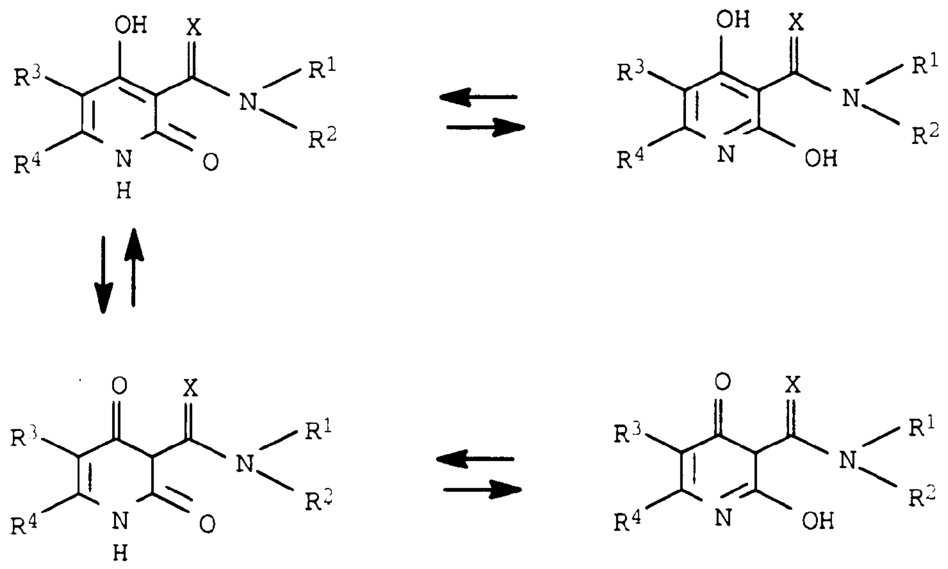

Die Herstellung der Hydroxypyridoncarbonsäureamide I gelingt durch Umsetzung von Verbindungen der Formel II

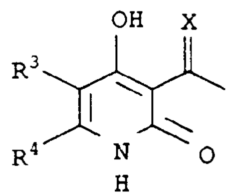

(II),

in der X, $R^3$ und $R^4$ die oben angegebenen Bedeutungen haben und Y ein Halogenatom, eine OH-Gruppe oder eine Alkoxygruppe bedeuten, mit einem Amin der Formel III,

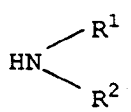

(III),

in der $R^1$ und $R^2$ die oben angegebenen Bedeutungen haben, unter geeigneten, allgemein bekannten Bedingungen.

Besonders vorteilhaft werden Alkylester (Y = O-Alkyl) z.B. Methyl- oder Ethylester mit den Aminen der Formel III umgesetzt. Diese Amidierung erfolgt im allgemeinen im Temperaturbereich von 50 - 250°C, vorzugsweise 110 - 160°C im Rührverfahren oder im Autoklaven.

Als Lösungsmittel eignen sich Alkohole wie Methanol, Ethanol, Propanol sowie Kohlenwasserstoffe wie Benzol oder Toluol, Wasser, Ether wie Diethylether oder Tetrahydrofuran, sowie aprotische dipolare Lösungsmittel wie Dimethylformamid, Dimethylsulfoxid oder Pyridin.

Es können auch Gemische dieser Stoffe als Lösungs- und Verdünnungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe, vorzugsweise das Amin, in einem Überschuß von 0,1 bis 10 Moläquivalenten zu verwenden.

Die Amidierung wird im allgemeinen bei Atmosphärendruck ausgeführt. Es kann jedoch, je nach Art des verwendeten Amins oder Lösungsmittels, vorteilhaft sein, die Umsetzung unter erhöhtem Druck, insbesondere unter autogen erhöhtem Druck in einem Autoklaven durchzuführen.

Die Herstellung der Ausgangsverbindungen der Formel II ist beschrieben in: J. Het. Chem. 20 (5), 1363

(1983); Synthesis (6), 479 (1988); Liebigs Ann. Chem. (3), 371 (1979); Synth. Commun. 7 (2) 149 (1977).

Die für die Umsetzungen benötigten Amine der Formel III sind bekannt, kommerziell erhältlich oder lassen sich nach allgemein bekannten chemischen Verfahren herstellen.

Im Hinblick auf die bestimmungsgemäße Verwendung der Hydroxypyridoncarbonsäureamide der allgemeinen Formel I kommen als Substituenten folgende Reste in Betracht:

$R^1$, $R^2$

Wasserstoff;

unverzweigtes oder verzweigtes $C_1$-$C_{25}$-Alkyl, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl, 1-Ethyl-2-methyl-propyl, Heptyl, 1-Methylheptyl, Octyl, Nonyl, Decyl, Undecyl, dodecyl, Tridecyl, Tetradecyl, Pentadecyl, Hexadecyl, Heptadecyl, Octadecyl, Nonadecyl, Arachidyl, Heneicosanyl, Docosyl, Tricosyl, Tetracosyl und Pentacosyl;

insbesondere $C_1$-$C_{15}$-Alkyl, beispielsweise $C_1$-$C_6$-Alkyl wie Methyl, Ethyl, 1-Methylethyl, 1-Methylpropyl, 2-Methylpropyl, 1,1-Dimethylethyl und 1,1-Dimethylpropyl;

unverzweigtes oder verzweigtes $C_3$-$C_{25}$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl, 10-Undecenyl, 9-Tetradecenyl, 9-Hexadecenyl, 6-Octadecenyl, 9-Octadecenyl, 11-Octadecenyl, 9,12-Octadecadienyl, 11-Eicosenyl, 13-Eicosenyl und 13-Docosenyl;

insbesondere $C_3$-$C_{15}$-Alkenyl, beispielsweise $C_3$-$C_6$-Alkenyl wie 2-Propenyl, 2-Butenyl, 3-Butenyl, 1-Methyl-2-propenyl, 2-Methyl-2-propenyl und 1-Methyl-2-butenyl;

$C_3$-$C_{25}$-Alkinyl, insbesondere $C_3$-$C_{15}$-Alkinyl, besonders bevorzugt $C_3$-$C_6$-Alkinyl, z.B. Propargyl, 2-Butinyl, 3-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;

$C_3$-$C_8$-Cycloalkyl wie Cyclopropyl, Cyclobutyl, Cyclopentyl, Cyclohexyl, Cycloheptyl, Cyclooctyl, insbesondere Cyclopropyl und Cyclohexyl;

$C_1$-$C_{25}$-Alkoxy, insbesondere $C_1$-$C_{15}$-Alkoxy, wobei der Alkylrest die vorstehend genannte Bedeutung hat, besonders bevorzugt $C_1$-$C_4$-Alkoxy wie Methoxy, Ethoxy, n-Propoxy, i-Propoxy, n-Butyloxy, iso-Butyloxy und tert-Butyloxy;

wobei diese Gruppen ein bis fünf Halogenatome wie Fluor, Chlor, Brom und Iod, vorzugsweise Fluor, Chlor und Brom, insbesondere Fluor und Chlor,

und/oder einen bis drei der folgenden Reste tragen können:

Cyano;

$C_1$-$C_5$-Alkyl- oder -Alkoxycarbonyl, wobei die darin vorliegenden Alkyl- und Alkoxystrukturelemente die voranstehend im einzelnen genannte Bedeutung haben, z.B. Methylcarbonyl oder Methoxycarbonyl;

$C_3$-$C_8$-Cycloalkyl wie Cyclopropyl, Cyclobutyl, Cyclopentyl, Cyclohexyl, Cycloheptyl, Cyclooctyl, insbesondere Cyclopropyl und Cyclohexyl;

$C_1$-$C_6$-Alkoxy wie Methoxy, Ethoxy, n-Propoxy, 2-Methylethoxy, n-Butoxy, 1-Methylpropoxy, 2-Methylpropoxy und 1,1-Dimethylethoxy, n-Pentoxy, n-Hexoxy, insbesondere $C_1$-$C_3$-Alkoxy wie Methoxy, Ethoxy, i-Propoxy;

$C_1$-$C_6$-Halogenalkoxy wie Chlormethoxy, Dichlormethoxy, Trichlormethoxy, Fluormethoxy, Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere $C_1$-$C_3$-Halogenalkoxy wie 2,2,2-Trifluorethyloxy und 2-Chlor-2,2-difluorethoxy;

$C_1$-$C_6$-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere $C_1$-$C_3$-Alkylthio wie Methylthio oder Ethylthio;
$C_1$-$C_6$-Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere $C_1$-$C_3$-Halogenalkylthio wie Trichlormethylthio;
Phenyl, wobei die Phenylreste ihrerseits eine bis fünf Halogenatome wie Fluor, Chlor, Brom und Iod, insbesondere Fluor und Chlor,
und/oder eine bis drei der folgenden Gruppen tragen können:
Cyano oder Nitro;
$C_1$-$C_4$-Alkyl, wie vorstehend genannt, insbesondere Methyl, Ethyl, Propyl, 1-Methylpropyl und 1,1-Dimethylpropyl;
$C_1$-$C_4$-Alkoxy, wie vorstehend genannt, insbesondere Methoxy und Ethoxy;
$C_1$-$C_4$-Halogenalkyl, wie vorstehend genannt, insbesondere Trifluormethyl;
$C_1$-$C_4$-Halogenalkoxy, wie vorstehend genannt, insbesondere Trifluormethoxy;
einen 5- bis 6-gliedrigen heterocyclischen, aliphatischen oder aromatischen Rest, enthaltend
ein bis drei Heteroatome, ausgewählt aus der Gruppe Sauerstoff, Schwefel oder Stickstoff, wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrrolyl, 3-Pyrazolyl, 4-Pyrazolyl, 5-Pyrazolyl, 2-Pyridyl, 4-Pyridyl, 4-Pyridyl, Oxa-2,4-diazolyl, Oxa-3,4-diazolyl, Thia-2,4-diazolyl, Thia-3,4-diazolyl und Triazolyl,
wobei diese heterocyclischen bzw. heteroaromatischen Substituenten ihrerseits einen oder zwei der folgenden Substituenten tragen können:
Halogen wie Fluor, Chlor, Brom und Iod, insbesondere Fluor und Chlor,
$C_1$-$C_3$-Alkyl oder $C_1$-$C_3$-Alkoxy, wie vorstehend genannt;
$R^3$
Wasserstoff;
Halogen wie Fluor, Chlor, Brom und Iod, insbesondere Fluor und Chlor;
Nitro;
$C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio und $C_1$-$C_6$-Halogenalkylthio, jeweils wie vorstehend für $R^1$ und $R^2$ im einzelnen genannt;
$R^4$
$C_1$-$C_{10}$-Alkyl, insbesondere $C_1$-$C_6$-Alkyl wie vorstehend für $R^1$ oder $R^2$ im einzelnen aufgeführt, z.B. Methyl, Ethyl, tert.-Butyl;
$C_2$-$C_{10}$-Alkenyl oder $C_2$-$C_{10}$-Alkinyl, insbesondere $C_2$-$C_6$-Alkenyl oder $C_2$-$C_6$-Alkinyl wie vorstehend für $R^1$ im einzelnen aufgeführt;
$C_1$-$C_{10}$-Alkoxy oder $C_1$-$C_{10}$-Alkylthio, insbesondere $C_1$-$C_6$-Alkoxy und $C_1$-$C_6$-Alkylthio wie vorstehend $R^1$ oder $R^2$ genannt, z.B. Methoxy, Ethoxy, Methylthio, Ethylthio;
Phenyl oder ein 5- bis 6-gliedriger heterocyclischer, aliphatischer oder aromatischer Rest, enthaltend ein bis drei Heteroatome, ausgewählt aus der Gruppe Sauerstoff, Schwefel oder Stickstoff, wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazolyl, Thia-2,4-diazolyl, Thia-3,4-diazolyl und Triazolyl, wobei der Phenylrest und die heterocyclischen bzw. heteroaromatischen Reste einen oder zwei der folgenden Substituenten tragen können:
Halogen wie Fluor, Chlor, Brom und Jod, insbesondere Fluor und Chlor,
$C_1$-$C_3$-Alkyl, $C_1$-$C_3$-Halogenalkyl, $C_1$-$C_3$-Halogenalkoxy, $C_1$-$C_3$-Alkoxy, jeweils wie vorstehend genannt,
Phenyl oder einen der bei $R^4$ genannten heterocyclischen oder heteroaromatischen Substituenten, wobei diese Reste ihrerseits substituiert sein können durch Halogen, $C_1$-$C_3$-Alkyl, $C_1$-$C_3$-Halogenalkyl, $C_1$-$C_3$-Halogenalkoxy, $C_1$-$C_3$-Alkoxy, jeweils wie vorstehend genannt;
Beispielsweise seien folgende Reste genannt: 4-Chlorphenyl, 4-Methylphenyl, 4-Trifluormethylphenyl, 4-Methoxyphenyl, 4-Trifluormethoxyphenyl.

Als Salze der Verbindungen I kommen landwirtschaftlich brauchbare Salze, beispielsweise Alkalimetallsalze, insbesondere das Kalium- oder Natriumsalz, Erdalkalimetallsalze, insbesondere Calcium-, Magnesium- oder Bariumsalz, Mangan-, Kupfer-, Zink- oder Eisensalze sowie Ammonium-, Phosphonium-, Tetraalkylammoniumsalze, Benzyltrialkylammoniumsalze, Trialkylsulfoniumsalze oder Trialkylsulfoxoniumsalze in Betracht.

Beispiele für besonders bevorzugte Verbindungen der allgemeinen Formel I sind in der folgenden Tabelle zusammengestellt:

Tabelle: Verbindungen der Struktur I

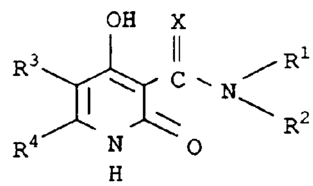

| $R^1$ | $R^2$ | $R^3$ | $R^4$ | X |
|---|---|---|---|---|
| H | Methoxy | H | $CH_3$ | O |
| H | Ethoxy | H | $CH_3$ | O |
| H | Propoxy | H | $CH_3$ | O |
| H | Benzyloxy | H | $CH_3$ | O |
| H | 2-Phenoxyethyl | H | $CH_3$ | O |
| H | tert. Butyl | H | $CH_3$ | O |
| H | tert. Butyl | H | F | O |
| H | tert. Butyl | H | $C_2H_5$ | O |
| H | iso-Propyl | H | $C_2H_5$ | O |
| H | iso-Propyl | H | $CH_3$ | O |
| H | iso-Propyl | H | F | O |
| H | tert. Butyl | Methyl | $CH_3$ | O |
| H | iso-Propyl | Methyl | $CH_3$ | O |
| H | tert. Butyl | H | $CF_3$ | O |
| H | 1,1-Dimethyl-2-propenyl | H | $CH_3$ | O |
| H | 2-Propinyl | H | $CH_3$ | O |
| H | 1-Methyl-2-propinyl | H | $CH_3$ | O |
| H | 1,1-Dimethyl-2-propinyl | H | $CH_3$ | O |
| H | Benzyl | H | $CH_3$ | O |
| H | 1-Methyl-phenylmethyl | H | $CH_3$ | O |
| H | 1,1-Dimethylphenylmethyl | H | $CH_3$ | O |
| H | 2-Phenylethyl | H | $CH_3$ | O |
| H | 2-Methylthioethyl | H | $CH_3$ | O |
| H | 1-Methyl-2-methylthioethyl | H | $CH_3$ | O |

| $R^1$ | $R^2$ | $R^3$ | $R^4$ | X |
|---|---|---|---|---|
| H | 2-Fluorethyl | H | $CH_3$ | O |
| H | 2-Fluor-1-methylethyl | H | $CH_3$ | O |
| H | 1,1-Dimethyl-2-fluorethyl | H | $CH_3$ | O |
| H | 2-Chlorethyl | H | $CH_3$ | O |
| H | 2-Chlor-1-methyl-ethyl | H | $CH_3$ | O |
| H | 2-Chlor-1,1-dimethylethyl | H | $CH_3$ | O |
| H | 2-Cyanoethyl | H | $CH_3$ | O |
| H | 2-Cyano-1,1-dimethylethyl | H | $CH_3$ | O |
| H | 2-Chlorbenzyl | H | H | O |
| H | 4-Methoxybenzyl | H | H | O |
| H | iso-Propyl | H | $CF_3$ | O |
| H | tert. Butyl | H | Phenyl | O |
| H | iso-Propyl | H | Phenyl | O |
| H | tert. Butyl | H | Methoxy | O |
| H | tert. Butyl | $NO_2$ | H | O |
| H | iso-Propyl | $NO_2$ | H | O |
| H | tert. Butyl | $CH_3$ | H | O |
| H | tert. Butyl | H | 1,1,2,2-Tetra-fluoro-ethoxy | O |
| H | tert. Butyl | Cl | H | O |
| H | iso-Propyl | Cl | H | O |
| H | tert. Butyl | $OCH_3$ | $OCH_3$ | O |
| H | tert. Butyl | $CH_3$ | $CF_3$ | O |
| H | tert. Butyl | H | - | O |
| H | tert. Butyl | Br | $CH_3$ | O |
| H | tert. Butyl | $NO_2$ | Phenyl | O |
| $CH_3$ | Methyl | H | $CH_3$ | O |
| $CH_3$ | Ethyl | H | $CH_3$ | O |
| H | Methoxy | H | $CH_3$ | S |
| H | Ethoxy | H | $CH_3$ | S |
| H | Propoxy | H | $CH_3$ | S |
| H | Benzyloxy | H | $CH_3$ | S |
| H | 2-Phenoxyethyl | H | $CH_3$ | S |
| H | tert. Butyl | H | $CH_3$ | S |
| H | tert. Butyl | H | F | S |
| H | tert. Butyl | H | $C_2H_5$ | S |

| $R^1$ | $R^2$ | $R^3$ | $R^4$ | X |
|---|---|---|---|---|
| H | iso-Propyl | H | $C_2H_5$ | S |
| H | iso-Propyl | H | $CH_3$ | S |
| H | iso-Propyl | H | F | S |
| H | tert. Butyl | Methyl | $CH_3$ | S |
| H | iso-Propyl | Methyl | $CH_3$ | S |
| H | tert. Butyl | H | $CF_3$ | S |
| H | 1,1-Dimethyl-2-propenyl | H | $CH_3$ | S |
| H | 2-Propinyl | H | $CH_3$ | S |
| H | 1-Methyl-2-propinyl | H | $CH_3$ | S |
| H | 1,1-Dimethyl-2-propinyl | H | $CH_3$ | S |
| H | Benzyl | H | $CH_3$ | S |
| H | 1-Methyl-phenylmethyl | H | $CH_3$ | S |
| H | 1,1-Dimethylphenylmethyl | H | $CH_3$ | S |
| H | 2-Phenylethyl | H | $CH_3$ | S |
| H | 2-Methylthioethyl | H | $CH_3$ | S |
| H | 1-Methyl-2-methylthioethyl | H | $CH_3$ | S |
| H | 2-Fluorethyl | H | $CH_3$ | S |
| H | 2-Fluor-1-methylethyl | H | $CH_3$ | S |
| H | 1,1-Dimethyl-2-fluorethyl | H | $CH_3$ | S |
| H | 2-Chlorethyl | H | $CH_3$ | S |
| H | 2-Chlor-1-methyl-ethyl | H | $CH_3$ | S |
| H | 2-Chlor-1,1-dimethylethyl | H | $CH_3$ | S |
| H | 2-Cyanoethyl | H | $CH_3$ | S |
| H | 2-Cyano-1,1-dimethylethyl | H | $CH_3$ | S |
| H | 2-Chlorbenzyl | H | H | S |
| H | 4-Methoxybenzyl | H | H | S |
| H | iso-Propyl | H | $CF_3$ | S |
| H | tert. Butyl | H | Phenyl | S |
| H | iso-Propyl | H | Phenyl | S |
| H | tert. Butyl | H | Methoxy | S |
| H | tert. Butyl | $NO_2$ | H | S |
| H | iso-Propyl | $NO_2$ | H | S |
| H | tert. Butyl | $CH_3$ | H | S |
| H | tert. Butyl | H | 1,1,2,2-Tetra-fluoro-ethoxy | S |
| H | tert. Butyl | Cl | H | S |

| $R^1$ | $R^2$ | $R^3$ | $R^4$ | X |
|---|---|---|---|---|
| H | iso-Propyl | Cl | H | S |
| H | tert. Butyl | $OCH_3$ | $OCH_3$ | S |
| H | tert. Butyl | $CH_3$ | $CF_3$ | S |
| H | tert. Butyl | H | - | S |
| H | tert. Butyl | Br | $CH_3$ | S |
| H | tert. Butyl | $NO_2$ | Phenyl | S |
| $CH_3$ | Methyl | H | $CH_3$ | S |
| $CH_3$ | Ethyl | H | $CH_3$ | S |

Die Verbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, vernetzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 bis 100 %, vorzugsweise 95 bis 100 % (nach NMR-Spektrum) eingesetzt.

Die erfindungsgemäßen Verbindungen I können beispielsweise wie folgt formuliert werden:

I. Man vermischt 90 Gew.-Teilen der Verbindung Nr. 1 mit 10 Gew.-Teilen N-Methyl-$\alpha$-pyrrolidon und erhält eine Lösung, die zur Anwendung in Form kleinster Tropfen geeignet ist;

II. 20 Gew.-Teile der Verbindung Nr. 1 werden in einer Mischung gelöst, die aus 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl besteht. Durch Ausgießen und feines Verteilen der Lösung in 100.000 Gew.-Teilen Wasser erhält man eine wäßrige Dispersion, die 0,02 Gew.-% des Wirkstoffs enthält.

III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.

IV. 20 Gew.-Teile des Wirkstoffs Nr. 1 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.

V. 20 Gew.-Teile des Wirkstoffs Nr. 1 werden mit 3 Gew.-Teilen des Natriumsalzes der Diisobutylnaphtalin-α-sulfonsäure, 1 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.-% des Wirkstoffs enthält.

VI. 3 Gew.-Teilen des Wirkstoffs Nr. 1 werden mit 97 Gew.-Teilen feinteiligem Kaolin vermischt. Man erhält auf diese Weise ein Stäubemittel, das 3 Gew.-% des Wirkstoffs enthält.

VII. 30 Gew.-Teilen des Wirkstoffs Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gew.-Teile des Wirkstoffs Nr. 1 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenolsulfonsäureharnstoff-formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 1 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |

| Botanischer Name | Deutscher Name |
|---|---|
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohne |
| Vitis vinifera | Weinrebe |
| Zea mays | Mais |

Zur Verbreiterung des Wirkungsspektrums und zur Erzielung synergistischer Effekte können die Hydroxypyridoncarbonsäureamide I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Herstellung von 4-Hydroxy-6-methylpyrid-2-on-3-carbonsäure-n-butylamid (Beispiel Nr. 4 der Tabelle 1)

9,85 g (0,05 mol) 4-Hydroxy-6-methylpyrid-2-on-3-carbonsäureethylester wurden mit 4,6 g (0,06 mol) n-Butylamin in 60 ml Ethanol in einem Miniautoklaven 8 Stunden bei 150°C unter Eigendruck gerührt. Nach dem Abkühlen werden die Kristalle abgesaugt, mit Petrolether gewaschen und getrocknet.

Ausbeute: 8,5 g (75,9 % der Theorie) Fp.: 158°C.

In entsprechender Weise wurden unter Abwandlung der Ausgangsstoffe weitere Verbindungen I hergestellt. Die so erhaltenen Verbindungen sind in der nachstehenden Tabelle 1 mit physikalischen Angaben aufgeführt.

Tabelle 1: Hydroxypyridon-3-carbonsäureamide I

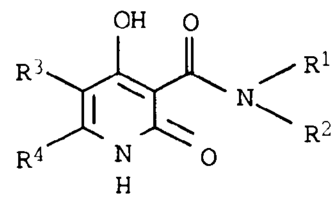

| Nr. | $R^1$ | $R^2$ | $R^3$ | $R^4$ | Schmelzpunkt [°C] |
|---|---|---|---|---|---|
| 1 | H | tert.-Butyl | H | $CH_3$ | 241 |
| 2 | H | iso-Propyl | H | $CH_3$ | 203 |
| 3 | H | n-Propyl | H | $CH_3$ | 202-203 |
| 4 | H | n-Butyl | H | $CH_3$ | 158 |
| 5 | H | Methyl | H | $CH_3$ | > 260 |
| 6 | H | Ethyl | H | $CH_3$ | 227 |
| 7 | H | 1-Methyl-1-Ethyl-propyl | H | $CH_3$ | 233 |
| 8 | H | 2-Methyl-n-butyl- | H | $CH_3$ | 170 |
| 9 | H | 1-Methyl-n-butyl- | H | $CH_3$ | 193 |
| 10 | $CH_3$ | tert.-butyl | H | $CH_3$ | |
| 11 | $CH_3$ | $CH_3$ | H | $CH_3$ | |

| Nr. | $R^1$ | $R^2$ | $R^3$ | $R^4$ | Schmelzpunkt [°C] |
|---|---|---|---|---|---|
| 12 | $C_2H_5$ | $C_2H_5$ | H | $CH_3$ | 95-105 |
| 13 | H | i-Propyl | H | $CF_3$ | 197-203 |
| 14 | H | tert.-Butyl | H | $CF_3$ | 200-203 |
| 15 | H | tert.-Butyl | Br | $CH_3$ | 252-254 |
| 16 | H | i-Propyl | Br | $CH_3$ | 256 |
| 17 | H | i-Propyl | Cl | $CH_3$ | 221 |
| 18 | H | tert.-Butyl | Cl | $CH_3$ | 257 |
| 19 | H | n-Propyl | $NO_2$ | $C_2H_5$ | |
| 20 | H | i-Propyl | $NO_2$ | $CH_3$ | 246 |
| 21 | H | tert.-Butyl | $NO_2$ | $CH_3$ | 270 |
| 22 | H | tert.-Butyl | H | 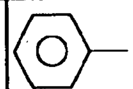 | 183 |
| 23 | H | i-Propyl | H | 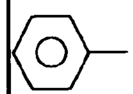 | 208 |
| 24 | H | 1,3-Dimethylpropyl | H | $CH_3$ | 215 |
| 25 | H | 1-n-propyl-n-butyl | H | $CH_3$ | 203 |
| 26 | H | 1-Ethyl-n-propyl | H | $CH_3$ | 205 |
| 27 | H | 1,1-Dimethyl-n-propyl | H | $CH_3$ | 252 |
| 28 | H | 1,1,2-Trimethylpropyl | H | $CH_3$ | 251 |

| Nr. | $R^1$ | $R^2$ | $R^3$ | $R^4$ | Schmelz-punkt [°C] |
|---|---|---|---|---|---|
| 29 | H | 2,2-Dimethylpropyl | H | $CH_3$ | 217 |
| 30 | H | 3-Methyl-1(2'-methyl-propyl)-butyl | H | $CH_3$ | 225-235 |
| 31 | H | 2-Ethyl-n-butyl- | H | $CH_3$ | 180 |
| 32 | H | 2,4,4-Trimethyl-pent-2-yl | H | $CH_3$ | 215 |
| 33 | H | 1-Methyl-1-ethyl-n-pentyl | H | $CH_3$ | 178-179 |
| 34 | H | n-Pentyl | H | $CH_3$ | 174 |
| 35 | H | 1-Methyl-2-methoxyethyl | H | $CH_3$ | 187 |
| 36 | H | 1-Ethyl-2-methoxyethyl | H | $CH_3$ | 186 |
| 37 | H | n-Heptyl | H | $CH_3$ | 138 |
| 38 | H | n-Nonyl | H | $CH_3$ | 132-142 |
| 39 | H | n-Undecyl | H | $CH_3$ | 116-117 |
| 40 | H | n-Pentadecyl | H | $CH_3$ | 120-121 |
| 41 | H | 1-Methylpropyl | H | $CH_3$ | 143-148 |
| 42 | H | Cyclopropyl | H | $CH_3$ | 247 |
| 43 | H | 2,6-Difluorbenzyl | H | $CH_3$ | 260 |
| 44 | H | (+)-1-Methylbenzyl | H | $CH_3$ | 203 |
| 45 | H | (-)-1-Methylbenzyl | H | $CH_3$ | 203-205 |
| 46 | H | (D)-1-Methylbenzyl | H | $CH_3$ | 214 |

| Nr. | $R^1$ | $R^2$ | $R^3$ | $R^4$ | Schmelzpunkt [°C] |
|---|---|---|---|---|---|
| 47 | t-Butyl | H | H | 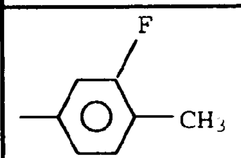 | 178 |
| 48 | t-Butyl | H | H | 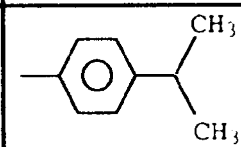 | 143-146 |
| 49 | t-Butyl | H | H | 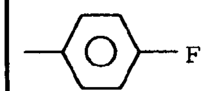 | 218-225 |
| 50 | iso-Propyl | H | $CH_3$ | $CH_3$ | 232 |
| 51 | tert.-Butyl | H | $CH_3$ | $CH_3$ | 258 |
| 52 | 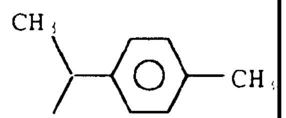 | H | $CH_3$ | $CH_3$ | 202-203 |
| 53 | H | i-Propyl | n-Butyl | $CH_3$ | 180 |
| 54 | H | t-Butyl | n-Butyl | $CH_3$ | 213-220 |
| 55 | H | t-Butyl | Ethyl | $CH_3$ | 238 |

| Nr. | $R^1$ | $R^2$ | $R^3$ | $R^4$ | Schmelzpunkt [°C] |
|---|---|---|---|---|---|
| 56 | H | i-Propyl | H | 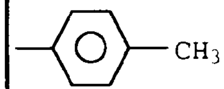 | 243 |
| 57 | H | i-Propyl | H | 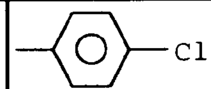 | 223 |
| 58 | H | i-Propyl | H | $n-C_4H_9$ | 183 |
| 59 | H | i-Propyl | H | 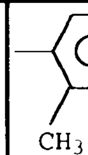 | 193-195 |
| 60 | H | t-Butyl | H | 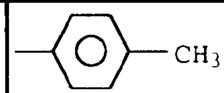 | 213-214 |
| 61 | H | t-Butyl | H | 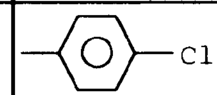 | 226 |
| 62 | H | t-Butyl | H | $n-C_4H_9$ | 209 |
| 63 | H | t-Butyl | H | 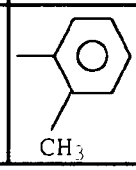 | 178 |

| Nr. | $R^1$ | $R^2$ | $R^3$ | $R^4$ | Schmelz-punkt [°C] |
|---|---|---|---|---|---|
| 64 | H | 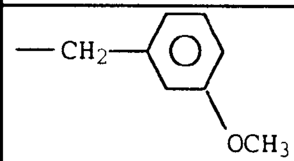 | H | $CH_3$ | 215 |
| 65 | H | 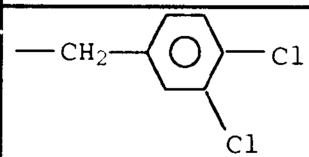 | H | $CH_3$ | 226 |
| 66 | H | 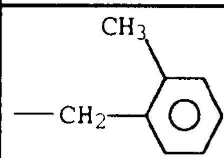 | H | $CH_3$ | 195 |
| 67 | H | 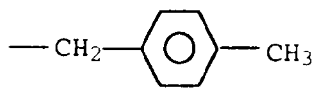 | H | $CH_3$ | 233-235 |
| 68 | H | 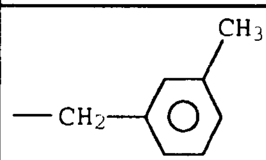 | H | $CH_3$ | 178 |

| Nr. | $R^1$ | $R^2$ | $R^3$ | $R^4$ | Schmelzpunkt [°C] |
|---|---|---|---|---|---|
| 69 | 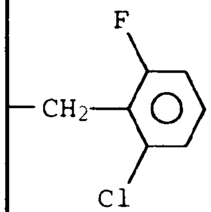 | H | H | $CH_3$ | 254 |
| 70 | 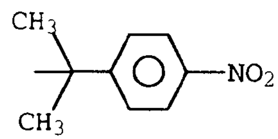 | H | H | $CH_3$ | 252 |
| 71 | 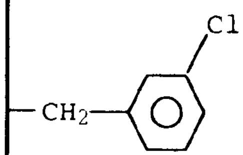 | H | H | $CH_3$ | 230 |
| 72 | 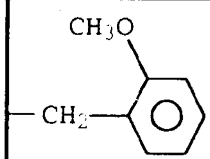 | H | H | $CH_3$ | 210 |
| 73 | H | iso-Butyl | H | $CH_3$ | 170 |
| 74 | H | sec.-Butyl | H | $CH_3$ | 204 |

| Nr. | $R^1$ | $R^2$ | $R^3$ | $R^4$ | Schmelzpunkt [°C] |
|---|---|---|---|---|---|
| 75 | H | 2,6-Diethylbenzyl | H | $CH_3$ | 232-233 |
| 76 | t-Butyl | H | H | Benzyl | 192 |
| 77 | t-Butyl | H | H | 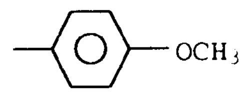 | 170 |
| 78 | t-Butyl | H | H | i-Propyl | 218 |
| 79 | 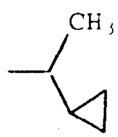 | H | H | 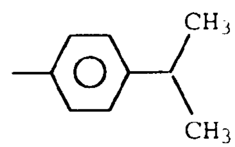 | 147 |
| 80 | 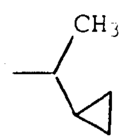 | H | H | 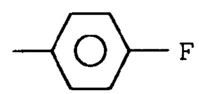 | 170-173 |
| 81 | 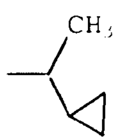 | H | H | Benzyl | 122-125 |
| 82 | i-Propyl | H | H | 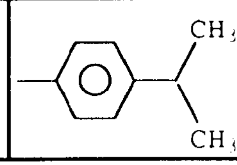 | 213 |

| Nr. | $R^1$ | $R^2$ | $R^3$ | $R^4$ | Schmelz-punkt [°C] |
|---|---|---|---|---|---|
| 83 | i-Propyl | H | H | 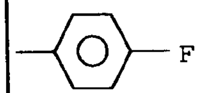 | 204-206 |
| 84 | i-Propyl | H | H | Benzyl | 192 |
| 85 | i-Propyl | H | H | 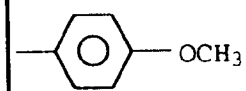 | 182 |
| 86 | i-Propyl | H | H | i-Propyl | 165 |
| 87 | i-Propyl | H | H | $CH_3$ | 278 |
| 88 | H | t-Butyl | H | $CH_3$ | 207-210 |
| 89 | H | 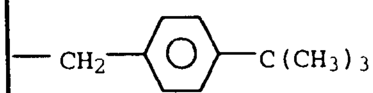 | H | $CH_3$ | 211 |
| 90 | H | 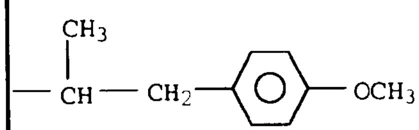 | H | $CH_3$ | 172 |

| Nr. | $R^1$ | $R^2$ | $R^3$ | $R^4$ | Schmelzpunkt [°C] |
|---|---|---|---|---|---|
| 91 | H | 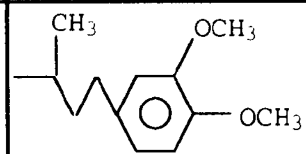 | H | $CH_3$ | 162-166 |
| 92 | H | 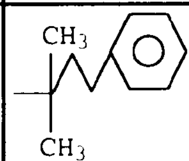 | H | $CH_3$ | 157-159 |
| 93 | 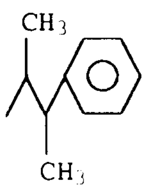 | H | H | $CH_3$ | 183 |
| 94 | 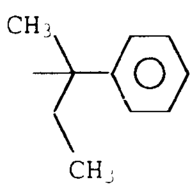 | H | H | $CH_3$ | 148-150 |

| Nr. | $R^1$ | $R^2$ | $R^3$ | $R^4$ | Schmelz-punkt [°C] |
|---|---|---|---|---|---|
| 95 | 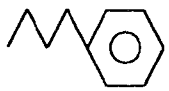 | H | H | $CH_3$ | 198 |
| 96 | 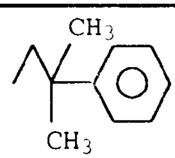 | H | H | $CH_3$ | 167-170 |
| 97 | 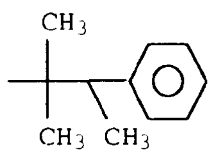 | H | H | $CH_3$ | 176-177 |
| 98 | 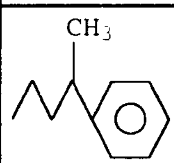 | H | H | $CH_3$ | 102-103 |

| Nr. | $R^1$ | $R^2$ | $R^3$ | $R^4$ | Schmelzpunkt [°C] |
|---|---|---|---|---|---|
| 99 | 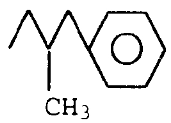 | H | H | $CH_3$ | 118-120 |
| 100 | 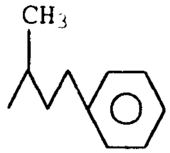 | H | H | $CH_3$ | 135-138 |
| 101 | 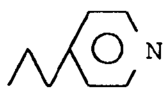 | H | H | $CH_3$ | 233-236 |
| 102 | 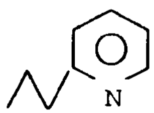 | H | H | $CH_3$ | 213 |
| 103 | i-Butyl | H | H | $CH_3$ | 170 |
| 104 | p-Chlorbenzyl | H | H | $CH_3$ | 238 |
| 105 | 2,4-Dichlorbenzyl | H | H | $CH_3$ | 267 |
| 106 | 2-Fluorbenzyl | H | H | $CH_3$ | 182 |
| 107 | 3-Fluorbenzyl | H | H | $CH_3$ | 207-210 |

| Nr. | $R^1$ | $R^2$ | $R^3$ | $R^4$ | Schmelzpunkt [°C] |
| --- | --- | --- | --- | --- | --- |
| 108 | 4-tert-Butyloxybenzyl | H | H | $CH_3$ | 175-177 |
| 109 | p-Fluorbenzyl | H | H | $CH_3$ | 232-235 |
| 110 | 2,5-Dimethylbenzyl | H | H | $CH_3$ | 174 |
| 111 | 2,4-Dimethylbenzyl | H | H | $CH_3$ | 203 |
| 112 | 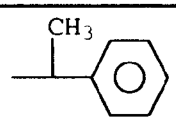 (-)-Form | H | $CH_3$ | $CH_3$ | 250 |
| 113 | H | 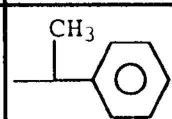 (-)-Form | H | $CH_3$ | 146-148 |
| 114 | H | 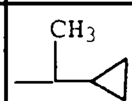 | H | $CH_3$ | 207 |

Anwendungsbeispiele

Die herbizide Wirkung der Verbindungen I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 3 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Echinochloa crus-galli | Hühnerhirse |
| Centaurea cyanus | Kornblume |

Mit 3 kg/ha a.S. im Nachauflaufverfahren eingesetzt, erzielt man mit den Beispielen 1, 7, 9, 29 und 31 sehr gute Wirkung gegen Echinochloa crus-galli; die Verbindungen 1, 2, 7, 14, 24, 26-28, 34, 35 und 74 bekämpfen Centaurea cyanus sehr wirksam.

## Patentansprüche

1. Hydroxypyridoncarbonsäureamide der allgemeinen Formel I,

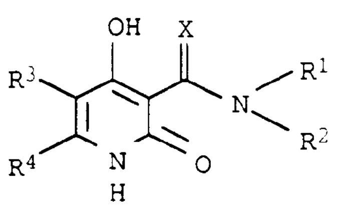

(I),

in der die Substituenten folgende Bedeutungen haben:

| | |
|---|---|
| X | Sauerstoff oder Schwefel; |
| $R^1$, $R^2$ | eunabhängig voneinander Wasserstoff, verzweigtes oder unverzweigtes $C_1$-$C_{25}$-Alkyl, $C_3$-$C_{25}$-Alkenyl, $C_3$-$C_{25}$-Alkinyl, $C_3$-$C_8$-Cycloalkyl, $C_3$-$C_8$-Cycloalkyl-$C_1$-$C_{17}$-alkyl, $C_1$-$C_{25}$-Alkoxy, wobei die genannten organischen Reste substituiert sein können durch Halogen, Cyano, $C_1$-$C_5$-Alkylcarbonyl, $C_1$-$C_5$-Alkoxycarbonyl, $C_1$-$C_6$-Alkoxy, $C_1$-$C_6$-Halogenalkoxy, $C_1$-$C_6$-Alkylthio, $C_1$-$C_6$-Halogenalkylthio, gegebenenfalls substituiertes Phenyl, gegebenenfalls substituiertes Heterocyclyl oder durch einen gegebenenfalls substituierten heteroaromatischen Rest; |
| $R^3$ | Wasserstoff, $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen oder die Nitrogruppe; |
| $R^4$ | $C_1$-$C_{10}$-Alkyl, $C_2$-$C_{10}$-Alkenyl, $C_2$-$C_{10}$-Alkinyl, $C_3$-$C_{10}$-Cycloalkyl, $C_1$-$C_{10}$-Alkoxy, $C_1$-$C_{10}$-Al- |

kylthio, Phenyl oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten Reste gegebenenfalls substituiert sind durch Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Halogenalkylthio, gegebenenfalls substituiertes Phenyl oder einen gegebenenfalls substituierten heterocyclischen Rest wie vorstehend genannt;

und deren umweltverträgliche Salze,

ausgenommen 6-Methyl-4-hydroxy-pyrid-2-on-3-carbonsäureamid und 6-Methyl-4-hydroxy-pyrid-2-on-3-carbonsäure-tert-butylamid.

**2.** Hydroxypyridoncarbonsäureamide der Formel I gemäß Anspruch 1, in der X und die Reste $R^1$ bis $R^4$ folgende Bedeutungen haben:

X Sauerstoff oder Schwefel;

$R^1$ Wasserstoff;

$R^2$ verzweigtes oder unverzweigtes $C_1$-$C_{15}$-Alkyl, $C_3$-$C_{15}$-Alkenyl, $C_3$-$C_{15}$-Alkinyl, $C_1$-$C_{15}$-Alkoxy, $C_3$-$C_6$-Cycloalkyl, wobei die genannte Reste durch Halogen, $C_1$-$C_3$-Alkoxy, $C_1$-$C_3$-Halogenalkoxy oder gegebenenfalls substituiertes Phenyl substituiert sein können;

$R^3$ Wasserstoff, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Halogenalkylthio, Halogen oder die Nitrogruppe;

$R^4$ $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Alkoxy, $C_1$-$C_6$-Alkylthio, Phenyl, Heterocyclyl oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten Reste gegebenenfalls substituiert sind durch Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Halogenalkylthio, gegebenenfalls substituiertes Phenyl oder einen gegebenenfalls substituierten heterocyclischen Rest wie vorstehend genannt,

ausgenommen 6-Methyl-4-hydroxy-pyrid-2-on-3-carbonsäure-tert.-butylamid.

**3.** Hydroxypyridoncarbonsäureamide der Formel I, gemäß Anspruch 1, in der X und die Reste $R^1$ bis $R^4$ folgende Bedeutungen haben:

X Sauerstoff oder Schwefel;

$R^1$ Wasserstoff;

$R^2$ verzweigtes oder unverzweigtes $C_1$-$C_6$-Alkyl, $C_3$-$C_6$-Alkenyl, $C_3$-$C_6$-Alkinyl, $C_1$-$C_4$-Alkoxy;

$R^3$ Wasserstoff, $C_1$-$C_3$-Alkyl, $C_1$-$C_3$-Halogenalkyl, $C_1$-$C_3$-Alkoxy, Halogen oder die Nitrogruppe;

$R^4$ $C_1$-$C_6$-Alkyl oder Phenyl, die jeweils durch 1 bis 3 Reste ausgewählt aus der Gruppe Halogen, $C_1$-$C_3$-Alkoxy, $C_1$-$C_3$-Halogenalkoxy, $C_1$-$C_3$-Alkylthio, $C_1$-$C_3$-Halogenalkylthio oder gegebenenfalls substituiertes Phenyl substituiert sein können,

ausgenommen 6-Methyl-4-hydroxy-pyrid-2-on-3-carbonsäure-tert.-butylamid.

**4.** Verfahren zur Herstellung der Hydroxypyridoncarbonsäureamide der Formel I gemäß Anspruch 1, dadurch gekennzeichnet, daß man Verbindungen der Formel II,

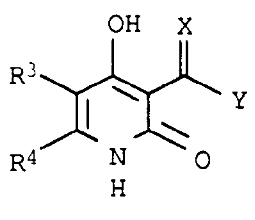

(II),

in der X, $R^3$ und $R^4$ die in Anspruch 1 angegebenen Bedeutungen haben und Y ein Halogenatom, eine OH-Gruppe oder eine Alkoxygruppe bedeuten, mit einem Amin der Formel III,

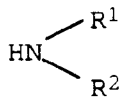

(III),

in der $R^1$ und $R^2$ die in Anspruch 1 angegebenen Bedeutungen haben, unter geeigneten, allgemein bekannten Bedingungen umsetzt.

5. Herbizide Mittel, enthaltend ein Hydroxypyridoncarbonsäureamid der Formel I gemäß Anspruch 1 und übliche inerte Zusatzstoffe.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Hydroxypyridoncarbonsäureamids der allgemeinen Formel I

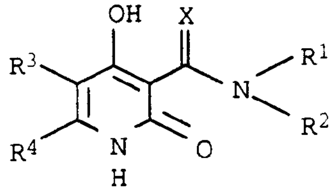

(I),

in der die Substituenten folgende Bedeutungen haben:

X Sauerstoff oder Schwefel;

$R^1$, $R^2$ unabhängig voneinander Wasserstoff, verzweigtes oder unverzweigtes $C_1$-$C_{25}$-Alkyl, $C_3$-$C_{25}$-Alkenyl, $C_3$-$C_{25}$-Alkinyl, $C_3$-$C_8$-Cycloalkyl, $C_3$-$C_8$-Cycloalkyl-$C_1$-$C_{17}$-alkyl, $C_1$-$C_{25}$-Alkoxy, wobei die genannten organischen Reste gegebenenfalls substituiert sein können durch Halogen, Cyano, $C_1$-$C_5$-Alkylcarbonyl, $C_1$-$C_5$-Alkoxycarbonyl, $C_1$-$C_6$-Alkoxy, $C_1$-$C_6$-Halogenalkoxy, $C_1$-$C_6$-Alkylthio, $C_1$-$C_6$-Halogenalkylthio, gegebenenfalls substituiertes Phenyl, gegebenenfalls substituiertes Heterocyclyl oder durch einen gegebenenfalls substituierten heteroaromatischen Rest;

$R^3$ Wasserstoff, $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen oder die Nitrogruppe;

$R^4$ $C_1$-$C_{10}$-Alkyl, $C_2$-$C_{10}$-Alkenyl, $C_2$-$C_{10}$-Alkinyl, $C_3$-$C_{10}$-Cycloalkyl, $C_1$-$C_{10}$-Alkoxy, $C_1$-$C_{10}$-Alkylthio, Phenyl oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten Reste gegebenenfalls substituiert sind durch Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Halogenalkylthio, gegebenenfalls substituiertes Phenyl oder einen gegebenenfalls substituierten heterocyclischen Rest wie vorstehend genannt;

behandelt.

## Claims

1. A hydroxypyridonecarboxamide of the formula I

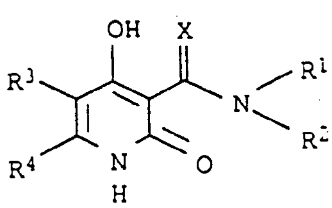

(I)

where
X is oxygen or sulfur;
$R^1$ and $R^2$ independently of one another are each hydrogen, branched or straight-chain $C_1$-$C_{25}$-alkyl, $C_3$-$C_{25}$-alkenyl, $C_3$-$C_{25}$-alkynyl, $C_3$-$C_8$-cycloalkyl, $C_3$-$C_8$-cycloalkyl-$C_1$-$C_{17}$-alkyl or $C_1$-$C_{25}$-alkoxy, and the stated organic radicals may be substituted by halogen, cyano, $C_1$-$C_5$-alkylcarbonyl, $C_1$-$C_5$-alkoxycarbonyl , $C_1$-$C_6$-alkoxy, $C_1$-$C_6$-haloalkoxy, $C_1$-$C_6$-alkylthio, $C_1$-$C_6$-haloalkylthio, unsubstituted or substituted phenyl or unsubstituted or substituted heterocyclyl or by an unsubstituted or substituted heteroaromatic radical;
$R^3$ is hydrogen, $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen or nitro;
$R^4$ is $C_1$-$C_{10}$-alkyl, $C_2$-$C_{10}$-alkenyl, $C_2$-$C_{10}$-alkynyl, $C_3$-$C_{10}$-cycloalkyl, $C_1$-$C_{10}$-alkoxy, $C_1$-$C_{10}$-alkylthio, phenyl or a 5 membered or 6-membered heterocyclic, aliphatic or aromatic radical containing from one to three hetero atoms selected from the group consisting of oxygen, sulfur and nitrogen, and the stated radicals may be substituted by halogen, $C_1$-$C_3$-alkyl, $C_1$-$C_3$-haloalkyl, $C_1$-$C_3$-alkoxy, $C_1$-$C_3$-haloalkoxy, $C_1$-$C_3$-haloalkylthio, unsubstituted or substituted phenyl or an unsubstituted or substituted heterocyclic radical as stated above,
or environmentally compatible salts thereof, with the exception of 6-methyl-4-hydroxypyrid-2-one-3-carboxamide and 6-methyl-4-hydroxypyrid-2-one-3-tert-butylcarboxamide.

2. A hydroxypyridonecarboxamide of the formula I as claimed in claim 1,
where
X is oxygen or sulfur;
$R^1$ is hydrogen;
$R^2$ is branched or straight-chain $C_1$-$C_{15}$-alkyl, $C_3$-$C_{15}$-alkenyl, $C_3$-$C_{15}$-alkynyl, $C_1$-$C_{15}$-alkoxy or $C_3$-$C_6$-cycloalkyl, and the stated radicals may be substituted by halogen, $C_1$-$C_3$-alkoxy, $C_1$-$C_3$-haloalkoxy or unsubstituted or substituted phenyl;
$R^3$ is hydrogen, $C_1$-$C_3$-alkyl, $C_1$-$C_3$-haloalkyl, $C_1$-$C_3$-alkoxy, $C_1$-$C_3$-haloalkoxy, $C_1$-$C_3$-alkylthio, $C_1$-$C_3$-haloalkylthio, halogen or nitro;
$R^4$ is $C_1$-$C_6$-alkyl, $C_2$-$C_6$-alkenyl, $C_2$-$C_6$-alkynyl, $C_3$-$C_6$-cycloalkyl, $C_1$-$C_6$-alkoxy, $C_1$-$C_6$-alkylthio, phenyl, heterocyclyl or a 5-membered or 6-membered heterocyclic, aliphatic or aromatic radical containing from one to three hetero atoms selected from the group consisting of oxygen, sulfur and nitrogen, and the stated radicals may be substituted by halogen, $C_1$-$C_3$-alkyl, $C_1$-$C_3$-haloalkyl, $C_1$-$C_3$-alkoxy, $C_1$-$C_3$-haloalkoxy, $C_1$-$C_3$-haloalkylthio, unsubstituted or substituted phenyl or an unsubstituted or substituted heterocyclic radical as stated above,
with the exception of 6-methyl-4-hydroxypyrid-2-one-3-tert-butylcarboxamide.

3. A hydroxypyridonecarboxamide of the formula I as claimed in claim 1,
where X is oxygen or sulfur;
$R^1$ is hydrogen;
$R^2$ is branched or straight-chain $C_1$-$C_6$-alkyl, $C_3$-$C_6$-alkenyl, $C_3$-$C_6$-alkynyl or $C_1$-$C_4$-alkoxy;
$R^3$ is hydrogen, $C_1$-$C_3$-alkyl, $C_1$-$C_3$-haloalkyl, $C_1$-$C_3$-alkoxy, halogen or nitro and
$R^4$ is $C_1$-$C_6$-alkyl or phenyl, each of which may be substituted by 1 to 3 radicals selected from the group consisting of halogen, $C_1$-$C_3$-alkoxy, $C_1$-$C_3$-haloalkoxy, $C_1$-$C_3$-alkylthio, $C_1$-$C_3$-haloalkylthio and unsubstituted or substituted phenyl,
with the exception of 6-methyl-4-hydroxypyrid-2-one-3-tert-butylcarboxamide.

4. A process for the preparation of a hydroxypyridonecarboxamide of the formula I as claimed in claim 1, wherein a compound of the formula II

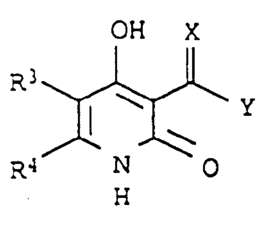

(II)

where X, $R^3$ and $R^4$ have the meanings stated in claim 1 and Y is halogen, OH or alkoxy is reacted with an amine of the formula III

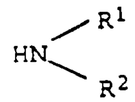

(III)

where $R^1$ and $R^2$ have the meanings stated in claim 1, under suitable, generally known conditions.

**5.** A herbicide containing a hydroxypyridone-carboxamide of the formula I as claimed in claim 1 and conventional inert additives.

**6.** A method for controlling undesirable plant growth, wherein the undesirable plants or their habitat is or are treated with a herbicidal amount of a hydroxypyridonecarboxamide of the formula I

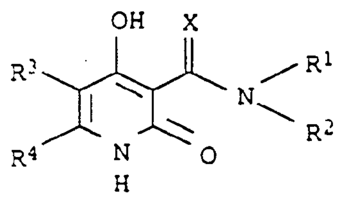

(I)

where
X is oxygen or sulfur;
$R^1$ and $R^2$ independently of one another are each hydrogen, branched or straight-chain $C_1$-$C_{25}$-alkyl, $C_3$-$C_{25}$-alkenyl, $C_3$-$C_{25}$-alkynyl, $C_3$-$C_8$-cycloalkyl, $C_3$-$C_8$-cycloalkyl-$C_1$-$C_{17}$-alkyl or $C_1$-$C_{25}$-alkoxy, and the stated organic radicals may be substituted by halogen, cyano, $C_1$-$C_5$-alkylcarbonyl, $C_1$-$C_5$-alkoxycarbonyl, $C_1$-$C_6$-alkoxy, $C_1$-$C_6$-haloalkoxy, $C_1$-$C_6$-alkylthio, $C_1$-$C_6$-haloalkylthio, unsubstituted or substituted phenyl or unsubstituted or substituted heterocyclyl or by an unsubstituted or substituted heteroaromatic radical;
$R^3$ is hydrogen, $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen or nitro;
$R^4$ is $C_1$-$C_{10}$-alkyl, $C_2$-$C_{10}$-alkenyl, $C_2$-$C_{10}$-alkynyl, $C_3$-$C_{10}$-cycloalkyl, $C_1$-$C_{10}$-alkoxy, $C_1$-$C_{10}$-alkylthio, phenyl or a 5-membered or 6-membered heterocyclic, aliphatic or aromatic radical containing from one to three hetero atoms selected from the group consisting of oxygen, sulfur and nitrogen, and the stated radicals may be substituted by halogen, $C_1$-$C_3$-alkyl, $C_1$-$C_3$-haloalkyl, $C_1$-$C_3$-alkoxy, $C_1$-$C_3$-haloalkoxy, $C_1$-$C_3$-haloalkylthio, unsubstituted or substituted phenyl or an unsubstituted or substituted heterocyclic radical as stated above.

## Revendications

**1.** Amides d'acide hydroxypyridoncarboxylique de la formule générale I

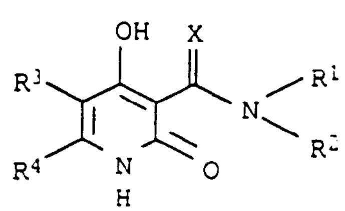

(I),

dans laquelle les substituants ont les significations suivantes :

X oxygène ou soufre
$R^1$, $R^2$ indépendamment les uns des autres, hydrogène, alkyle en C1-C25, alcényle en C3-C25, alcynile en C3-C25, cycloalkyle en C3-C8, cycloalkyle en C3-C8-alkyle en C1-C17, alcoxy en

C1-C25 ramifiés ou non ramifiés, les restes organiques indiqués pouvant être substitués par halogène, cyano, alkyle en C1-C5-carbonyle, alcoxy en C1-C5-carbonyle, alcoxy en C1-C5, halogénalcoxy en C1-C6, alkylthio en C1-C6, halogénalkyl en C1-C6-thio, phényle éventuellement substitué, hétérocycle éventuellement substitué ou par un reste hétéroaromatique éventuellement substitué

$R^1$ hydrogène, alkyle en C1-C6, halogénalkyle en C1-C6, alcoxy en C1-C6, halogénalcoxy en C1-C6, alkyle en C1-C6-thio, halogénalkyl en C1-C6-thio, halogène ou le groupe nitro

$R^4$ alkyle en C1-C10, alcényle en C2-C10, alcinyle en C2-C10, cycloalkyle en C3-C10, alcoxy en C1-C10, alkyle en C1-C10-thio, phényle ou un reste hétérocyclique à 5 ou 6 chaînons, aliphatique ou aromatique, contenant un à trois hétéroatomes choisis dans le groupe oxygène, soufre ou azote, les restes indiqués étant éventuellement substitués par halogène alkyle en C1-C3, halogénalkyle en C1-C3, alcoxy en C1-C3, halogénalcoxy en C1-C3, halogénalkyl en C1-C3-thio, phényle éventuellement substitué ou un reste hétérocyclique éventuellement substitué tel qu'indiqué précédemment

et leurs sels acceptables pour l'environnement exceptés de l'amide d'acide [6-méthyl-4-hydroxy-pyrid-2-on-3-carboxylique] et le tert-butylamide d'acide [6-méthyl-4-hydroxy-pyrid-2-on-3-carboxylique].

**2.** Amides d'acide hydroxypyridoncarboxylique de la formule générale I dans laquelle x et les restes $R^1$ à $R^4$ ont les significations suivantes

X oxygène ou soufre

$R^1$ hydrogène

$R^2$ alkyle en C1-C15, alcényle en C3-C15, alcinyle en C3-C15, alcoxy en C1-C15, cycloalkyle en C3-C6 ramifiés ou non ramifiés, les restes indiqués pouvant être substitués par halogène, alcoxy en C1-C3, halogénalcoxy en C1-C3 ou phényle éventuellement substitué

$R^3$ hydrogène, alkyle en C1-C3, halogénalkyle en C1-C3, alcoxy en C1-C3, halogénalcoxy en C1-C3, alkyle en C1-C3-thio, halogénalkyle en C1-C3-thio, halogène ou le groupe nitro

$R^4$ alkyle en C1-C6, alcényle en C2-C6, alcinyle en C2-C6, cycloalkyle en C3-C6, alcoxy en C1-C6, alkylthio en C1-C6, phényle, hétérocycle ou un reste hétéroxyclique à 5 ou 6 chaînons, aliphatique ou aromatique contenant un à trois hétéroatomes du groupe du soufre oxygène, soufre ou azote, les restes indiqués étant éventuellement substitués par halogène, alkyle en C1-C3, halogénalkyle en C1-C3, alcoxy en C1-C3, halogénalcoxy en C1-C3, halogénalkyl en C1-C3-thio, phényle éventuellement substitué ou un reste hétérocyclique éventuellement substitué tel qu'indiqué précédemment

excepté le tert-butylamide d'acide 6-méthyl-4-hydroxy-pyrid-2-on-3-carboxylique.

**3.** Amides d'acide hydroxypyridoncarboxylique de la formule générale I dans laquelle x et les restes R1 à R4 ont les significations suivantes

X oxygène ou soufre

$R^1$ hydrogène

$R^2$ alkyle en C1-C6, alcényle en C3-C6, alcinyle en C3-C6, alcoxy en C1-C4, ramifiés ou non ramifiés

$R^3$ hydrogène, alkyle en C1-C3, halogénalkyle en C1-C3, alcoxy en C1-C3, halogène ou le groupe nitro

$R^4$ alkyle en C1-C6 ou phényle qui peuvent chacun être substitués par 1 à 3 restes choisis dans le groupe halogène, alcoxy en C1-C3, halogénalcoxy en C1-C3, alkyle en C1-C3-thio, halogénalkyle en C1-C3-thio ou phényle éventuellement substitué

excepté le tert-butylamide d'acide 6-méthyl-4-hydroxy-pyrid-2-on-3-carboxylique

**4.** Procédé de préparation d'amides d'acide hydroxypyridoncarboxylique de la formule générale I selon la revendication 1, caractérisé par le fait que l'on fait réagir, dans des conditions appropriées, généralement connues, des composés de la formule II

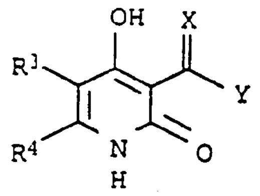

(II),

dans laquelle X, $R^3$ et $R^4$ ont les significations données dans la revendication 1 et Y représente un atome d'halogène, un groupe OH ou un groupe alcoxy, avec une amine de la formule III

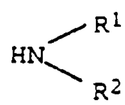

(III),

dans laquelle $R^1$ et $R^2$ ont les significations données dans la formule I.

5. Herbicide, contenant une amides d'acide hydroxypyridoncarboxylique de la formule générale I selon la revendication 1 et les additifs usuels.

6. Procédé pour lutter contre la croissance des plantes indésirables, caractérisé par le fait qu'on traite les plantes indésirables et/ou leur biotope avec une quantité à activité herbicide d'un amide d'acide hydroxypyridoncarboxylique de la formule générale I

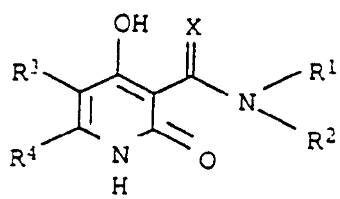

(I),

dans laquelle les substituants ont les significations suivantes :

| | |
|---|---|
| X | oxygène ou soufre |
| $R^1$, $R^2$ | indépendamment l'un de l'autre, hydrogène, alkyle en C1-C25, alcényle en C3-C25, alcynile en C3-C25, cycloalkyle en C3-C8, cycloalkyle en C3-C8, alkyle en C1-C17, alcoxy en C1-C25 ramifiés ou non ramifiés les restes organiques indiqués pouvant être substitués par halogène, cyano, alkyle en C1-C5-carbonyle, alcoxy en C1-C5-carbonyle, alcoxy en C1-C5, halogénalcoxy en C1-C6, alkylthio en C1-C6, halogénalkyl en C1-C6-thio, phényle éventuellement substitué, hétérocycle éventuellement substitué, ou par un reste hétéroaromatique éventuellement substitué |
| $R^1$ | hydrogène, alkyle en C1-C6, halogénalkyle en C1-C6, alcoxy en C1-C6, halogénalcoxy en C1-C6, alkyle en C1-C6-thio, halogénalkyl en C1-C6-thio, halogène ou le groupe nitro |
| $R^4$ | alkyle en C1-C10, alcényle en C2-C10, alcinyle en C2-C10, cycloalkyle en C3-C10, alcoxy en C1-C10, alkyle en C1-C10-thio, phényle ou un reste hétérocyclique à 5 ou 6 chaînons, aliphatique ou aromatique, contenant un à trois hétéroatomes choisis dans le groupe oxygène soufre ou azote, les restes indiqués étant éventuellement substitués par halogène alkyle en C1-C3, halogénalkyle en C1-C3, alcoxy en C1-C3, halogénalcoxy en C1-C3, halogénalkyl en C1-C3-thio, phényle éventuellement substitué ou un reste hétérocyclique éventuellement substitué tel qu'indiqué précédemment. |